# EUROPEAN PATENT APPLICATION

(11) **EP 4 134 103 A1**
(43) Date of publication of application: **15.02.2023**
(21) Application number: 21784847.2
(22) Date of filing: 24.03.2021
(51) Int. Cl.: A61K 47/32, A61K 9/16, A61K 31/4422, A61K 31/522, A61K 31/525, A61K 47/10, A61K 47/26, A61K 47/38, A61P 3/02, A61P 9/12, A61P 11/00

(54) **BINDER**

(30) Priority: 10.04.2020 JP 2020071295
(71) Applicant: Japan Vam & Poval Co., Ltd., Sakai-shi, Osaka 592-8331 (JP)
(72) Inventor: KAWADA Shotaro, Sakai-shi, Osaka 592-8331 (JP); KAWANISHI Masatoshi, Sakai-shi, Osaka 592-8331 (JP)
(74) Representative: Lederer & Keller Patentanwälte Partnerschaft mbB
(86) International application number: PCT/JP2021/012432
(87) International publication number: WO 2021/205886

(57) **Abstract**

A novel binder is provided. The binder comprises a polyvinyl alcohol-based polymer having an average saponification value of 96.0 mol% or more.

## Description

### TECHNICAL FIELD

The present invention relates to a binder and others.

### BACKGROUND ART

Various polymers, such as hydroxypropylmethyl cellulose, are used as binders in pharmaceutical preparations, and polyvinyl alcohol (hereinafter also referred to as PVA) is also occasionally used for this purpose.

### SUMMARY OF INVENTION

### TECHNICAL PROBLEM

An object of the present invention is to provide a novel binder.

Another object of the present invention is to provide a novel preparation.

Yet another object of the present invention is to provide a method for producing a novel preparation.

### SOLUTION TO PROBLEM

As mentioned above, PVA is occasionally used as a binder in pharmaceutical preparations, but a type of PVA having a relatively high saponification value (e.g., 90 mol% or more) is not used because of its low water solubility.

Under such circumstances, the present inventors sought to use a PVA having a high saponification value as a binder in pharmaceutical preparations.

After intensive studies to achieve the above-mentioned objects, the present inventors found that a polyvinyl alcohol-based polymer having a high saponification value (in particular, 96.0 mol% or more) can be used as a binder.

The present inventors also found that using a polyvinyl alcohol-based polymer having a high saponification value (in particular, 96.0 mol% or more) as a base in pharmaceutical preparations can provide an excellent sustained-release property. The present inventors further conducted a great deal of examination and then completed the present invention.

That is, the present invention relates to the following.
[1] A binder comprising a polyvinyl alcohol-based polymer (1) having an average saponification value of 96.0 mol% or more.
[2] A sustained-release base comprising a polyvinyl alcohol-based polymer (1) having an average saponification value of 96.0 mol% or more.
[3] A granulated material comprising the binder according to the above [1] or the base according to the above [2].
[4] A solid preparation comprising a polyvinyl alcohol-based polymer (1) having an average saponification value of 96.0 mol% or more.
[5] The preparation according to the above [4], wherein the preparation is a sustained-release preparation.
[6] The preparation according to the above [4] or [5], wherein the preparation is an oral preparation.
[7] A solid preparation comprising the granulated material according to the above [3] and a polyvinyl alcohol-based polymer, wherein a 4 mass% aqueous solution of the polyvinyl alcohol-based polymer has a viscosity of 15.0 mPa·s or more as measured according to JIS K 6726, and/or wherein the polyvinyl alcohol-based polymer has an average saponification value of 65.0 to 90.0 mol%.
[8] The solid preparation according to the above [7], further comprising a low-substituted hydroxypropyl cellulose.
[9] The binder, base, granulated material, or preparation according to any one of the above [1] to [8], wherein the polyvinyl alcohol-based polymer (1) has an average polymerization degree of 200 or more.
[10] The binder, base, granulated material, or preparation according to any one of the above [1] to [9], wherein a 4 mass% aqueous solution of the polyvinyl alcohol-based polymer (1) has a viscosity of 3.0 to 30 mPa·s as measured according to JIS K 6726.
[11] The granulated material or preparation according to any one of the above [3] to [10], further comprising one or more fillers selected from lactose, mannitol, and crystalline cellulose.
[12] A method for producing a granulated material, comprising performing granulation using the binder or base according to any one of the above [1], [2], [9], and [10].
[13] A method for granulation using the binder or base according to any one of the above [1], [2], [9], and [10].
[14] A method for producing a solid preparation, comprising the step of bringing an aqueous and/or water-based solution containing the binder or base according to any one of the above [1], [2], [9], and [10] into contact with a substance to be mixed with the binder or base.
[15] A method for producing a solid preparation, comprising the step of directly compressing a mixture containing the granulated material according to any one of the above [3] and [9] to [11] and a polyvinyl alcohol-based polymer, wherein a 4 mass% aqueous solution of the polyvinyl alcohol-based polymer has a viscosity of 15.0 mPa·s or more as measured according to JIS K 6726, and/or wherein the polyvinyl alcohol-based polymer has an average saponification value of 65.0 to 90.0 mol%.
[16] The method according to the above [15], wherein the mixture further contains a low-substituted hydroxypropyl cellulose.
[17] Use of a polyvinyl alcohol-based polymer (1) having an average saponification value of 96.0 mol% or more as a binder or sustained-release base.
[18] A method for binding a substance of interest using a polyvinyl alcohol-based polymer (1) having an average saponification value of 96.0 mol% or more.
[19] A method for improving the sustained-release property of a solid preparation (or solid formulation), the method comprising using a polyvinyl alcohol-based polymer (1) having an average saponification value of 96.0 mol% or more.

### ADVANTAGEOUS EFFECTS OF INVENTION

In one aspect of the present invention, a novel binder is provided.

In one aspect of the present invention, a novel sustained-release base is provided.

In one aspect of the present invention, an agent (such as a binder or sustained-release base) that is useful in the production of solid preparations is provided. For example, the agent of the present invention can be used to form a granulated material (such as granules) as a component of solid preparations (such as tablets) so that an advantageous solid preparation in terms of hardness and sustained-release property can be efficiently provided.

With one embodiment of the agent, a solid preparation having an excellent sustained-release property is provided. Such a preparation has an excellent sustained-release property, and therefore excellent sustained release can be achieved even when a smaller amount of PVA is used.

With one embodiment of the agent, a solid preparation having a high hardness is provided. Such a preparation has an excellent formability because it does not readily fall apart after shaping or molding.

In one aspect of the present invention, a novel preparation (particularly, a solid preparation) is provided.

In one aspect of the present invention, a method for producing a novel preparation is provided.

### BRIEF DESCRIPTION OF DRAWINGS

Fig. 1 is a graph showing the time course of the dissolution rate in Examples 1 to 5.
Fig. 2 is a graph showing the time course of the dissolution rate in Examples 6 to 8.
Fig. 3 is a graph showing the time course of the dissolution rate in Examples 9 to 11.
Fig. 4 is a graph showing the time course of the dissolution rate in Reference Example 1.
Fig. 5 is a graph showing the time course of the dissolution rate in Reference Example 2.

### DESCRIPTION OF EMBODIMENTS

### Agent

The agent of the present invention comprises a specific type of polyvinyl alcohol-based polymer (1) (may be referred to as a PVA-based polymer, PVA, etc.).

The agent may function, for example, as either a binder or a sustained-release base, or as both a binder and a sustained-release base.

### Polyvinyl alcohol-based polymer

In the present invention, the polyvinyl alcohol-based polymer usually may be a saponified product of a vinyl ester-based polymer (a polymer at least composed of a vinyl ester as a polymerizable component).

The average saponification value of the PVA-based polymer (1) is usually 96.0 mol% or more, and preferably 96.5 mol% or more (e.g., 97.0 mol% or more, 97.5 mol% or more, or 98.0 mol% or more) from the perspective of ease of granulation and the sustained-release property of the preparation.

For the upper limit, the average saponification value of the PVA-based polymer (1) is, for example, 99.9 mol% or less (e.g., 99.8 mol% or less), preferably 99.7 mol% or less (e.g., 99.6 mol% or less), and more preferably 99.5 mol% or less.

These upper and lower limits may be combined to set an appropriate range (e.g., 96.0 to 99.9 mol%) for the average saponification value of the PVA-based polymer (1) (the same applies to the others). All combinations of these upper and lower limits are applicable.

The method for measuring the average saponification value of the PVA-based polymer (1) is not particularly limited, and for example, the method for measuring the saponification value specified in JIS K 6726 can be used.

The average polymerization degree of the PVA-based polymer (1) is, for example, 100 or more (e.g., 110 or more, 120 or more, 130 or more, or 140 or more), preferably 150 or more (e.g., 160 or more, 170 or more, 180 or more, or 190 or more), more preferably 200 or more (e.g., 210 or more, 220 or more, 230 or more, or 240 or more), and particularly preferably 250 or more (e.g., 260 or more, 270 or more, 280 or more, or 290 or more) from the perspective of ease of granulation and the sustained-release property of the preparation.

For the upper limit, the average polymerization degree of the PVA-based polymer (1) is, for example, 3000 or less (e.g., 2800 or less), preferably 2500 or less (e.g., 2300 or less), and more preferably 2000 or less (e.g., 1800 or less).

These upper and lower limits may be combined to set an appropriate range (e.g., 150 to 2000) for the average polymerization degree of the PVA-based polymer (1) (the same applies to the others). All combinations of these upper and lower limits are applicable.

The method for measuring the average polymerization degree of the PVA-based polymer (1) is not particularly limited, and for example, the method for measuring the average polymerization degree specified in JIS K 6726 can be used.

The viscosity of a 4 mass% aqueous solution of the PVA-based polymer (1) (as measured according to JIS K 6726) is, for example, 2.5 mPa·s or more (e.g., 2.7 mPa·s or more, 3.0 mPa·s or more, or 3.3 mPa·s or more), preferably 3.5 mPa·s or more (e.g., 3.7 mPa·s or more, 4.0 mPa·s or more, or 4.3 mPa·s or more), and more preferably 4.5 mPa·s or more (e.g., 4.7 mPa·s or more, 5.0 mPa·s or more, or 5.3 mPa·s or more) from the perspective of ease of granulation (e.g., fast granulation and easy production of larger-size granular powder) and the sustained-release property of the preparation.

For the upper limit, the viscosity of a 4 mass% aqueous solution of the PVA-based polymer (1) (as measured according to JIS K 6726) is, for example, 50 mPa·s or less (e.g., 48 mPa·s or less, 45 mPa·s or less, or 42 mPa·s or less), preferably 40 mPa·s or less (e.g., 38 mPa·s or less, 35 mPa·s or less, or 32 mPa·s or less), and more preferably 30 mPa·s or less (e.g., 28 mPa·s or less, 25 mPa·s or less, or 22 mPa·s or less).

These upper and lower limits may be combined to set an appropriate range (e.g., 3 to 30 mPa·s) for the viscosity of a 4 mass% aqueous solution of the PVA-based polymer (1) (the same applies to the others). All combinations of these upper and lower limits are applicable.

The PVA-based polymer (1) used may be obtained commercially or synthesized. The method for producing the PVA-based polymer is not particularly limited, and known methods, for example, saponification of a polymer containing a vinyl ester monomer as a polymerizable component (vinyl ester-based polymer), may be used.

The vinyl ester monomer is not particularly limited, and examples include fatty acid vinyl esters [e.g., C₁₋₂₀ fatty acid vinyl esters (e.g., C₁₋₁₆ alkanoic acid vinyl esters) such as vinyl formate, vinyl acetate, vinyl propionate, vinyl butyrate, vinyl caprylate, vinyl versatate, and vinyl monochloroacetate], and aromatic carboxylic acid vinyl esters [e.g., vinyl arenecarboxylates (e.g., C₇₋₁₂ arene carboxylic acid vinyl esters) such as vinyl benzoate].

A single kind of vinyl ester monomer or a combination of two or more kinds of vinyl ester monomers may be used.

The vinyl ester monomer preferably at least comprises a fatty acid vinyl ester (e.g., C₁₋₁₀ alkanoic acid vinyl esters such as vinyl formate, vinyl acetate, vinyl propionate, and vinyl butyrate). In particular, it is industrially preferable that the vinyl ester monomer comprises vinyl acetate.

The vinyl ester-based polymer has a vinyl ester unit, and if necessary, may have an additional monomer unit (a monomer capable of copolymerizing with vinyl ester monomers) (in other words, the vinyl ester-based polymer may be modified with an additional monomer).

The additional monomer is not particularly limited, and examples include, but are not limited to, α-olefins (e.g., ethylene, propylene, etc.), (meth)acrylic acid esters [e.g., (meth)acrylic acid alkyl esters such as methyl (meth)acrylate, ethyl (meth)acrylate, butyl (meth)acrylate, and 2-ethylhexyl (meth)acrylate], unsaturated amides [e.g., (meth)acrylamide, diacetone acrylamide, N-methylolacrylamide, etc.], unsaturated acids {e.g., unsaturated acids [e.g., (meth)acrylic acid, crotonic acid, maleic acid, itaconic acid, fumaric acid, etc.], unsaturated acid esters [unsaturated acid esters other than (meth)acrylic acid esters, e.g., alkyl (methyl, ethyl, propyl, etc.) esters, etc.], unsaturated acid anhydrides (maleic anhydride, etc.), salts of unsaturated acids [e.g., alkali metal salts (e.g., sodium salts, potassium salts, etc.), ammonium salts, etc.], etc.}, glycidyl group-containing monomers [e.g., allyl glycidyl ethers, glycidyl (meth)acrylate, etc.], sulfonic group-containing monomers (e.g., 2-acrylamide-2-methylpropane sulfonic acid, salts thereof, etc.), phosphate group-containing monomers [e.g., acid phosphoxy ethyl (meth)acrylate, acid phosphoxy propyl (meth)acrylate, etc.], vinyl ethers (e.g., alkyl vinyl ethers), allyl alcohols, etc.

A single kind of additional monomer or a combination of two or more kinds of additional monomers may be used.

In the case where the additional monomer is contained as a polymerizable component in the vinyl ester-based polymer, the amount of the additional monomer in the polymerizable components may be, for example, 50 mass% or less, 30 mass% or less, 20 mass% or less, or 10 mass% or less.

The amount of the vinyl ester monomer in the polymerizable components is, for example, 50 mass% or more, preferably 70 mass% or more, more preferably 90 mass% or more and may be 100 mass%.

In the PVA-based polymer (1), some vinyl alcohol units may be modified by a reaction, such as acetalization, etherification, acetoacetylization, or cationization.

The method for polymerizing the polymerizable components (e.g., the polymerizable components including the vinyl ester monomer such as vinyl acetate) is not particularly limited, and examples include known polymerization methods such as block polymerization, solution polymerization, suspension polymerization, and emulsion polymerization. Among them, solution polymerization using methanol as a solvent is industrially preferred. In the solution polymerization, known initiators such as peroxide initiators and azo initiators can be used, and the polymerization degree of the vinyl ester-based polymer can be adjusted by varying the feed ratio of the polymerizable components and methanol and the polymerization yield. For the production of the PVA-based polymer, commercial vinyl ester-based polymers (such as polyvinyl acetate resin) can be used as a starting material.

The method for saponifying the vinyl ester-based polymer (e.g., polyvinyl acetate) can be a conventional saponification method using an alkaline or acid catalyst. In particular, industrially preferred is alcoholysis, which is performed by adding an alkali such as sodium hydroxide to a solution of the vinyl ester-based polymer (e.g., polyvinyl acetate) in methanol or in a mixed solvent of methanol, water, methyl acetate, etc. and stirring the mixture for cleavage of the acyl group (e.g., acetyl group) of the vinyl ester-based polymer (e.g., polyvinyl acetate).

After that, the obtained mass product, gelled product, or granular product is pulverized, and if needed, the alkali is neutralized; then the solid is separated from the liquid and dried to yield a PVA-based polymer.

A single kind of PVA-based polymer (1) or a combination of two or more kinds of PVA-based polymers (1) may be used in the agent of the present invention.

The agent of the present invention preferably does not contain any resin other than the PVA-based polymer (particularly, a thermoplastic resin).

The substance to be mixed with the agent of the present invention is not particularly limited and may be, for example, a pharmaceutical product, a quasi-pharmaceutical product, a food product, an agrochemical product, or the like. The substance to be mixed with the agent of the present invention may be an active ingredient, a nutrient (or nutritional ingredient), or the like, and may be a medicinal substance (such as an active pharmaceutical ingredient).

The substance to be mixed with the agent of the present invention may be an organic substance or an inorganic substance, a mixture thereof, or an organic-inorganic hybrid material.

The medicinal substance or the active ingredient (or the medicinal substance or the active ingredient contained in the substance to be mixed with the agent of the present invention) is not particularly limited. Examples of the medicinal substance include central nervous system drugs, cerebral metabolism improving agents, cerebral circulation improving agents, antiepileptics, sympathomimetics, cardiovascular drugs, respiratory drugs, gastrointestinal drugs, antibiotics, antiallergics, antihistamines, dental and stomatological preparations, cardiotonics, antiarrhythmics, diuretics, antipyretic, analgesic and anti-inflammatory agents, autonomic drugs, antidepressants, antipsychotics, anxiolytics, sedative-hypnotics, vasodilators, antihypertensives, vasoconstrictors, peripheral vasodilators, anticoagulants, lipid-lowering agents, choleretics, antimalarial agents, antidiarrheals, psychotropics, chemotherapeutics, diabetes drugs, osteoporosis drugs, skeletal muscle relaxants, analeptics, antispasmodics, antirheumatic drugs, hormone preparations, alkaloid narcotics, sulfa drugs, anti-gout preparations, and antineoplastic agents.

Examples of the central nervous system drug include diazepam, idebenone, aspirin, ibuprofen, paracetamol, naproxen, piroxicam, indomethacin, sulindac, lorazepam, nitrazepam, phenytoin, acetaminophen, ethenzamide, ketoprofen, chlordiazepoxide, and citicoline.

Examples of the cerebral metabolism improving agent include meclofenoxate hydrochloride.

Examples of the cerebral circulation improving agent include vinpocetine.

Examples of the antiepileptic include phenytoin, carbamazepine, and sodium valproate.

Examples of the sympathomimetic include isoproterenol hydrochloride.

Examples of the cardiovascular drug include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, propranolol hydrochloride, and alprenolol hydrochloride.

Examples of the respiratory drug include antitussives and expectorants, respiratory stimulants, and bronchodilators.

Examples of the antitussive and expectorant include noscapine hydrochloride, carbetapentane citrate, dextromethorphan, dextromethorphan hydrobromide, isoaminile citrate, dimemorfan phosphate, cloperastine hydrochloride, dextromethorphan hydrobromide, theophylline, potassium guaiacolsulfonate, guaifenesin, guaifenesin-codeine phosphate, and ambroxol hydrochloride.

Examples of the respiratory stimulant include levallorphan tartrate.

Examples of the bronchodilator include theophylline, salbutamol, and salbutamol sulfate.

Examples of the gastrointestinal drug include gastrointestinal agents, antacids, antiulcer agents, digestive enzyme preparations, laxatives, and antiemetics.

Examples of the gastrointestinal agent include stomachic digestive agents (e.g., diastase, saccharated pepsin, scopolia extract, cellulase AP3, lipase AP, and cinnamon oil) and intestinal regulators (e.g., berberine chloride, antibiotics-resistant lactic acid bacteria, and bifidobacteria).

Examples of the antacid include magnesium carbonate, sodium hydrogen carbonate, magnesium aluminometasilicate, synthetic hydrotalcite, precipitated calcium carbonate, and magnesium oxide.

Examples of the antiulcer agent include 5-aminosalicylic acid, lansoprazole, omeprazole, rabeprazole, cimetidine, famotidine, ranitidine, ranitidine hydrochloride, pirenzepine hydrochloride, and antiulcer benzimidazole drugs such as 2-[[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole.

Examples of the digestive enzyme preparation include pancreatin.

Examples of the laxative include bisacodyl.

Examples of the antiemetic include difenidol hydrochloride and metoclopramide.

Examples of the antibiotic include cephems such as cephalexin, amoxicillin, cefaclor, pivmecillinam hydrochloride, cefotiam hexetil hydrochloride, cefadroxil, cefixime, cefditoren pivoxil, cefteram pivoxil and cefpodoxime proxetil; synthetic antibacterial agents such as ampicillin, ciclacillin, nalidixic acid, and enoxacin; monobactams such as carumonam sodium; macrolides such as erythromycin; penems; and carbapenems.

Examples of the antiallergic include amlexanox and seratrodast.

Examples of the antihistamine include diphenhydramine hydrochloride, promethazine, promethazine hydrochloride, isothipendyl hydrochloride, and dl-chlorpheniramine maleate.

Examples of the dental and stomatological preparation include oxytetracycline, triamcinolone acetonide, chlorhexidine hydrochloride, and lidocaine.

Examples of the cardiotonic include digoxin and caffeine.

Examples of the arrhythmic include procainamide hydrochloride, propranolol hydrochloride, and pindolol.

Examples of the diuretic include caffeine, furosemide, isosorbide, and hydrochlorothiazide.

Examples of the antipyretic, analgesic and anti-inflammatory agent include acetaminophen, aspirin, ibuprofen, ethenzamide, diphenhydramine hydrochloride, dl-chlorpheniramine maleate, diclofenac sodium, dihydrocodeine phosphate, salicylamide, aminopyrine, noscapine, methylephedrine hydrochloride, phenylpropanolamine hydrochloride, serrapeptase, lysozyme chloride, tolfenamic acid, mefenamic acid, flufenamic acid, ketoprofen, indomethacin, bucolome, pentazocine, caffeine, anhydrous caffeine, sulpyrine, hydromorphone hydrochloride, and tapentadol hydrochloride.

Examples of the autonomic agent include dihydrocodeine phosphate and dl-methylephedrine hydrochloride, atropine sulfate, acetylcholine chloride, and neostigmine.

Examples of the vasodilator include nifedipine, carbocromen hydrochloride, molsidomine, and verapamil hydrochloride.

Examples of the antihypertensive include captopril, delapril hydrochloride, hydralazine hydrochloride, labetalol hydrochloride, manidipine hydrochloride, candesartan cilexetil, methyldopa, and perindopril erbumine.

Examples of the vasoconstrictor include phenylephrine hydrochloride.

Examples of the peripheral vasodilator include cinnarizine.

Examples of the anticoagulant include dicoumarol.

Examples of the lipid-lowering agent include cerivastatin sodium, simvastatin, pravastatin sodium, and atorvastatin calcium hydrate.

Examples of the choleretic include dehydrocholic acid and trepibutone.

Examples of the antimalarial agent include quinine hydrochloride.

Examples of the antidiarrheal include loperamide hydrochloride.

Examples of the psychotropic include chlorpromazine and reserpine.

Examples of the antidepressant include amphetamine, imipramine, maprotiline hydrochloride, and paroxetine hydrochloride.

Examples of the antipsychotic include paliperidone.

Examples of the anxiolytic include diazepam, alprazolam, and chlordiazepoxide.

Examples of the sedative-hypnotic include estazolam, diazepam, nitrazepam, perlapine, and phenobarbital sodium.

Examples of the chemotherapeutic include sulfamethizole.

Examples of the diabetes drug include glymidine sodium, glipizide, phenformin hydrochloride, buformin hydrochloride, metformin, metformin hydrochloride, tolbutamide, voglibose, pioglitazone hydrochloride, glibenclamide, and troglitazone.

Examples of the osteoporosis drug include ipriflavone.

Examples of the skeletal muscle relaxant include methocarbamol.

Examples of the analeptic include vitamins and their derivatives [e.g., vitamin A, vitamin B 1, fursultiamine, vitamin B2 (riboflavin), vitamin B6, vitamin B 12, vitamin C, vitamin D, vitamin E, vitamin K, calcium pantothenate, tranexamic acid, etc.], minerals (e.g., calcium, magnesium, iron, etc.), proteins, amino acids, oligosaccharides, and herbal medicines.

Examples of the antispasmodic include meclizine hydrochloride, dimenhydrinate, scopolamine hydrobromide, diphenhydramine hydrochloride, and papaverine hydrochloride.

Examples of the antirheumatic drug include methotrexate and bucillamine.

Examples of the hormone preparation include liothyronine sodium, dexamethasone sodium phosphate, prednisolone, oxendolone, and leuprorelin acetate.

Examples of the alkaloid narcotic include opium, morphine hydrochloride, ipecac, oxycodone hydrochloride, opium alkaloids hydrochloride, and cocaine hydrochloride.

Examples of the sulfa drug include sulfisomidine and sulfamethizole.

Examples of the anti-gout preparation include allopurinol and colchicine.

Examples of the antineoplastic agent include 5-fluorouracil, uracil, and mitomycin.

The substance to be mixed with the agent of the present invention may further contain an additional ingredient (an additive usually used in this field, for example, a filler, a disintegrant, a lubricant, an antiagglomerant, a solubilizer, etc.).

Examples of the filler include saccharides, such as sucrose, lactose, mannitol, and glucose, starch, crystalline cellulose, calcium phosphate, calcium sulfate, etc. Preferred are lactose, mannitol, crystalline cellulose, etc.

Examples of the disintegrant include low-substituted hydroxypropyl cellulose, carmellose or a salt thereof, croscarmellose sodium, sodium carboxymethyl starch, crospovidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer include organic acids, such as fumaric acid, succinic acid, malic acid, and adipic acid, etc.

One of these additives or two or more of them may be used. The amount of the additional ingredient (the additive) can be determined as appropriate according to, for example, the type of the drug.

A single kind of substance to be mixed with the agent of the present invention or a combination of two or more kinds of substances to be mixed with said agent may be used.

The substance to be mixed with the agent of the present invention may be a solid at ordinary temperature (e.g., 10 to 40°C).

The substance to be mixed with the agent of the present invention may be, for example, in the form of powder (or powder particles). The particle size of the powder (active ingredient, active ingredient powder, etc.) is not particularly limited, and the average particle diameter is, for example, about 500 µm or less (e.g., 5 to 400 µm), preferably about 300 µm or less, and more preferably about 100 µm or less (e.g., 10 to 80 µm).

The average particle diameter of the powder may be measured with a laser-type particle size distribution measuring instrument, etc.

### Granulated material, etc.

In the case where the agent (binder, sustained-release base) of the present invention is used for granulation, granulated materials (granular powder) can be obtained. That is, the granulated material usually comprises the agent of the present invention and a substance to be mixed therewith (such as powder particles).

A powdery granulated material (granular powder) may usually be in the form of granules (in a granular shape).

The particle size of the granulated material (e.g., granules) can be determined as appropriate according to the size of the substance to be mixed with the agent of the present invention, the granulation method, etc. The average particle diameter is, for example, about 700 µm or less (e.g., 30 to 600 µm), preferably about 400 µm or less, and more preferably about 300 µm or less (e.g., 50 to 200 µm).

The average particle diameter of the granulated material may be measured with a laser-type particle size distribution measuring instrument, etc.

The amount of the agent (or the polyvinyl alcohol-based polymer (1)) of the present invention in the granulated material can be selected from the range of, for example, about 0.1 to 50 mass%, may be 0.2 to 30 mass% (e.g., 0.3 to 15 mass%), and is preferably 0.5 to 10 mass% (e.g., 0.7 to 8 mass%), more preferably about 1 to 5 mass% (e.g., 1 to 3 mass%).

The amount of the agent (or the polyvinyl alcohol-based polymer (1)) of the present invention in the granulated material can be selected from the range of, for example, about 0.001 to 0.5 part by mass relative to 1 part by mass of the substance to be mixed with said agent, may be about 0.002 to 0.2 part by mass (e.g., 0.003 to 0.15 part by mass), and is preferably about 0.005 to 0.1 part by mass (e.g., 0.007 to 0.08 part by mass), more preferably about 0.01 to 0.05 part by mass (e.g., 0.01 to 0.03 part by mass).

In the case where the substance to be mixed with the agent of the present invention contains a filler, the amount of the filler in the granulated material can be selected from the range of, for example, about 50 to 99 parts by mass relative to 1 part by mass of the polyvinyl alcohol-based polymer (1), may be about 60 to 99 parts by mass (e.g., 65 to 98.5 parts by mass), and is preferably about 70 to 98 parts by mass (e.g., 75 to 97.5 parts by mass), more preferably about 80 to 97 parts by mass (e.g., 85 to 96.5 parts by mass).

The agent of the present invention is usually used by contact with the substance to be mixed therewith.

In particular, by using the agent of the present invention for granulation (bringing the agent of the present invention into contact with the substance to be mixed therewith), a granulated material can be produced.

The granulation method is not particularly limited, and dry granulation (e.g., powder (such as medicinal substance powder) and a binder are mixed, the powder mixture is compacted by applying a force with an apparatus called a roller compactor to form larger particles, and the particles are ground to produce granules) may be used. Typically, wet granulation may be used.

Wet granulation at least includes a step of bringing the agent (or the polyvinyl alcohol-based polymer (1)) of the present invention in a liquid form into contact with the substance to be mixed therewith (granulation step). In a typical granulation step, the agent (or the polyvinyl alcohol-based polymer (1)) of the present invention may be brought into contact with the substance to be mixed therewith in the presence of a solvent. More specifically, a dispersion or solution (in particular, an aqueous and/or water-based solution) containing the binder or sustained-release base may be brought into contact with (e.g., sprayed onto or added dropwise onto) the substance to be mixed therewith (in particular, powder particles) (e.g., solution addition method). Alternatively, a solvent (a solvent which can dissolve or disperse the agent of the present invention) may be brought into contact with (e.g., sprayed onto or added dropwise onto) a mixture of the agent and the substance to be mixed therewith (powder particles) (e.g., powder addition method).

The solvent is not particularly limited as long as it can dissolve or disperse the agent (or the polyvinyl alcohol-based polymer (1)) of the present invention. Usually, water or a mixed solvent (water-based solvent) of water and an organic solvent is suitable, and in particular, water or a water-based solvent (typically water) is preferably suitable. The organic solvent (water-miscible solvent) is not particularly limited, and examples include alcohols (e.g., ethanol).

In the wet granulation, the amount of the solvent used (e.g., the amount of water in an aqueous solution) is, for example, about 1 to 70 parts by mass (e.g., 3 to 60 parts by mass), preferably about 4 to 50 parts by mass (e.g., 5 to 45 parts by mass), and more preferably about 7 to 40 parts by mass (e.g., 8 to 35 parts by mass) relative to 100 parts by mass of the substance to be mixed with the agent of the present invention (powder particles).

Specific examples of the wet granulation include fluid bed granulation, high-speed stirring granulation, extrusion granulation, tumbling granulation, etc. In particular, fluid bed granulation, high-speed stirring granulation, etc. are suitable in the present invention.

Fluid bed granulation is performed, for example, in the following procedure: the substance to be mixed with the agent of the present invention (powder particles) is fed into a layer called a fluid bed; and while warm air flow is supplied from the bottom to fluidize the substance (powder particles), a solution or dispersion (in particular, an aqueous solution) containing the agent (or the polyvinyl alcohol-based polymer (1)) of the present invention is sprayed from the above to form granules. In fluid bed granulation, drying is performed concomitantly with granulation (a step of forming granules), and the time of drying can be saved after granulation.

For high-speed stirring granulation, a high-speed stirring granulator with a large blade called a blender and a small blade called a chopper inside of the apparatus is used, for example. In such high-speed stirring granulation, the substance to be mixed with the agent of the present invention (powder) is fed into the apparatus, and while the stirring blades are rotated, a solution or dispersion (in particular, aqueous solution) containing the agent (or the polyvinyl alcohol-based polymer (1)) of the present invention is sprayed or added dropwise to form granules. After granulation, drying is performed and thus finished granules are obtained.

The granulated material (e.g., granules) can be used in various ways according to the type of the substance to be mixed with the agent of the present invention, the desired application, etc.

For example, the granulated material may be directly used as granules (e.g., granules, granules in a capsule, etc.) or shaped or molded into any form. For example, the granulated material (granules) can be used as a material for tablets. In other words, tablets can contain the granulated material and can be produced from the granulated material by tableting.

In the tablet production process, a granulated material containing a medicinal substance or active ingredient may be tableted, or a granulated material that does not contain a medicinal substance or active ingredient may be tableted together with a medicinal substance or active ingredient.

The tablet contains at least the granulated material and optionally an additional ingredient. In other words, the granulated material may be tableted together with an additional ingredient.

The additional ingredient may be an additive usually used in this field (e.g., a disintegrant, a lubricant, an antiagglomerant, a solubilizer, etc.).

Examples of the disintegrant include low-substituted hydroxypropyl cellulose, carmellose or a salt thereof, croscarmellose sodium, sodium carboxymethyl starch, crospovidone, microcrystalline cellulose, microcrystalline cellulose-carmellose sodium, etc.

Examples of the lubricant or the antiagglomerant include talc, magnesium stearate, calcium stearate, colloidal silica, stearic acid, waxes, hydrogenated oils, polyethylene glycols, sodium benzoate, etc.

Examples of the solubilizer include organic acids, such as fumaric acid, succinic acid, malic acid, and adipic acid, etc.

One of these additives or two or more of them may be used.

The amount of the additive can be determined as appropriate according to, for example, the type of the ingredient contained in the tablet or in the granulated material.

The tablet may contain a PVA-based polymer other than the PVA-based polymer (1) (hereinafter simply referred to as a "PVA-based polymer (2)") as the additional ingredient from the perspective of the sustained-release property of the preparation.

The average saponification value of the PVA-based polymer (2) is, for example, 90.0 mol% or less (e.g., 89.5 mol% or less) and preferably 89.0 mol% or less (e.g., 88.5 mol% or less or 88.0 mol% or less) from the perspective of the sustained-release property of the preparation.

For the lower limit, the average saponification value of the PVA-based polymer (2) is, for example, 60.0 mol% or more (e.g., 62.0 mol% or more) and preferably 65.0 mol% or more (e.g., 68.0 mol% or more, 70.0 mol% or more, 75.0 mol% or more, or 80.0 mol% or more).

These upper and lower limits may be combined to set an appropriate range (e.g., 60.0 to 90.0 mol%) for the average saponification value of the PVA-based polymer (2) (the same applies to the others). All combinations of these upper and lower limits are applicable.

The method for measuring the average saponification value of the PVA-based polymer (2) is not particularly limited, and for example, the method for measuring the saponification value specified in JIS K 6726 can be used.

The average polymerization degree of the PVA-based polymer (2) is, for example, 200 or more (e.g., 300 or more, 400 or more, 500 or more, 600 or more, 700 or more, 800 or more, or 900 or more), preferably 1000 or more (e.g., 1200 or more, 1500 or more, or 1800 or more), and more preferably 2000 or more (e.g., 2100 or more or 2400 or more) from the perspective of the sustained-release property of the preparation.

For the upper limit, the average polymerization degree of the PVA-based polymer (2) is, for example, 5000 or less (e.g., 4900 or less), preferably 4500 or less (e.g., 4400 or less), and more preferably 3500 or less (e.g., 3400 or less).

These upper and lower limits may be combined to set an appropriate range (e.g., 1500 to 3500) for the average polymerization degree of the PVA-based polymer (2) (the same applies to the others). All combinations of these upper and lower limits are applicable.

The method for measuring the average polymerization degree of the PVA-based polymer (2) is not particularly limited, and for example, the method for measuring the average polymerization degree specified in JIS K 6726 can be used.

The viscosity of a 4 mass% aqueous solution of the PVA-based polymer (2) (as measured according to JIS K 6726) is, for example, 6.0 mPa·s or more (e.g., 8.0 mPa·s or more), preferably 10.0 mPa·s or more (e.g., 12.0 mPa·s or more), and particularly preferably 15.0 mPa·s or more (e.g., 18.0 mPa·s or more or 20.0 mPa·s or more) from the perspective of the sustained-release property of the preparation.

For the upper limit, the viscosity of a 4 mass% aqueous solution of the PVA-based polymer (2) (as measured according to JIS K 6726) is, for example, 300 mPa·s or less (e.g., 280 mPa·s or less), preferably 240 mPa·s or less (e.g., 220 mPa·s or less), and more preferably 100 mPa·s (e.g., 80 mPa·s or less).

These upper and lower limits may be combined to set an appropriate range (e.g., 15 to 240 mPa·s) for the viscosity of a 4 mass% aqueous solution of the PVA-based polymer (2) (the same applies to the others). All combinations of these upper and lower limits are applicable.

In a particular embodiment, a 4 mass% aqueous solution of the PVA-based polymer (2) has a viscosity of 15.0 mPa·s or more as measured according to JIS K 6726, and/or the PVA-based polymer (2) has an average saponification value of 65.0 to 90.0 mol%.

The production method and polymerizable component of the PVA-based polymer (2) are not particularly limited, and may be the same as those of the PVA-based polymer (1) as described above.

A single kind of PVA-based polymer (2) or a combination of two or more kinds of PVA-based polymers (2) may be used.

In addition, the use of an appropriate type of additive can provide the preparation with a better sustained-release property.

For example, the use of an appropriate type of disintegrant (e.g., low-substituted hydroxypropyl cellulose) can provide the preparation with a better sustained-release property.

For low-substituted hydroxypropyl cellulose, the degree of substitution (degree of etherification) is, for example, about 0.05 to 1.0 (e.g., 0.07 to 0.8) and preferably about 0.1 to 0.6 (e.g., 0.15 to 0.5).

The degree of substitution may be the average number of substituted hydroxyl groups per glucose unit in the hydroxypropyl cellulose.

In the low-substituted hydroxypropyl cellulose, the substitution (mass%) of hydroxypropoxy groups for hydroxyl groups may be 5 to 16 mass%.

A single kind of low-substituted hydroxypropyl cellulose or a combination of two or more kinds of low-substituted hydroxypropyl celluloses may be used.

The amount of the agent (or the polyvinyl alcohol-based polymer (1)) of the present invention in the tablet is, for example, about 0.5 to 10 mass%, preferably about 2 to 6 mass% (e.g., 1 to 5 mass%), more preferably about 1 to 3 mass% and may be 3 mass% or less (e.g., 1 to 2 mass% or 2 to 3 mass%).

The shape of the tablet is not particularly limited and may be a disk shape, a lens shape, a pole shape, or some other shape.

The size of the tablet is also not particularly limited and may be, for example, 3 mm or more (e.g., 4 to 15 mm, 5 to 12 mm, 8 to 11 mm, etc.) in diameter (maximum diameter).

The tableting method is not particularly limited, and conventional tableting methods can be employed.

The tablet may have a coat layer on the surface of the tablet.

The component of the coat layer in the coated tablet is not particularly limited, and examples include resin materials, such as cellulose resin (e.g., hydroxypropylmethyl cellulose, hydroxypropylmethylcellulose acetate succinate, etc.), and polyvinyl alcohol-based polymers.

### Preparation

The present invention also encompasses a solid preparation containing the polyvinyl alcohol-based polymer (1) described above.

The substance to be formulated into the preparation of the present invention may be a substance to be mixed with the agent of the present invention. The substance to be formulated into the preparation is usually in the form of powder particles.

In the preparation of the present invention, powder particles are preferably bound (or bonded) to each other via the polyvinyl alcohol-based polymer (1).

The preparation of the present invention may be, for example, a tablet, granule, or capsule preparation.

The preparation of the present invention can be produced by shaping or molding using the granulated material described above.

The preparation of the present invention is suitable for use as an oral preparation.

The preparation of the present invention may further contain an additional ingredient (e.g., an additional ingredient as exemplified above in the tablet).

The use of an appropriate type of additional ingredient can provide the preparation with a better sustained-release property.

The preparation of the present invention may contain, for example, a PVA-based polymer (2) as exemplified above and/or a disintegrant as exemplified above (such as a low-substituted hydroxypropyl cellulose as exemplified above) as the additional ingredient.

The amount of the PVA-based polymer (1) in the preparation is, for example, about 0.5 to 10 mass%, preferably about 2 to 6 mass% (e.g., 1 to 5 mass%), more preferably about 1 to 3 mass% and may be 3 mass% or less (e.g., 1 to 2 mass%, 2 to 3 mass%, etc.).

The amount of the PVA-based polymer (1) in the preparation can be selected from the range of, for example, about 0.001 to 0.5 part by mass relative to 1 part by mass of the substance to be mixed with the agent of the present invention, may be about 0.002 to 0.2 part by mass (e.g., 0.003 to 0.15 part by mass), and is preferably about 0.005 to 0.1 part by mass (e.g., 0.007 to 0.08 part by mass), more preferably about 0.01 to 0.05 part by mass (e.g., 0.01 to 0.03 part by mass).

The amount of the filler in the preparation can be selected from the range of, for example, about 50 to 99 parts by mass relative to 1 part by mass of the PVA-based polymer (1), may be about 60 to 99 parts by mass (e.g., 65 to 98.5 parts by mass), and is preferably about 70 to 98 parts by mass (e.g., 75 to 97.5 parts by mass), more preferably about 80 to 97 parts by mass (e.g., 85 to 96.5 parts by mass).

In the case where the preparation contains a PVA-based polymer (2), the amount of the PVA-based polymer (2) in the preparation is, for example, about 20 to 60 mass% (e.g., 22 to 58 mass%), preferably about 25 to 55 mass% (e.g., 27 to 52 mass%), and more preferably about 30 to 50 mass% (e.g., 33 to 48 mass%).

In the case where the preparation contains a low-substituted hydroxypropyl cellulose, the amount of the low-substituted hydroxypropyl cellulose in the preparation is, for example, about 1 to 30 parts by mass (e.g., 2 to 27 parts by mass), preferably about 3 to 25 parts by mass (e.g., 4 to 23 parts by mass), and more preferably about 5 to 20 parts by mass relative to 1 part by mass of the PVA-based polymer (1).

In the case where the preparation contains a PVA-based polymer (2) and a low-substituted hydroxypropyl cellulose, the mass ratio of the PVA-based polymer (2):the low-substituted hydroxypropyl cellulose in the preparation is, for example, about 30:70 to 90:10 (e.g., 33:67 to 87:13), preferably about 35:65 to 85:15 (e.g., 40:60 to 80:20), and more preferably about 43:57 to 78:22 (e.g., 45:55 to 75:25).

The preparation of the present invention may be a sustained-release preparation.

For the sustained-release preparation (or granulated material) of the present invention, the time taken for the dissolution rate of the active ingredient (e.g., an active ingredient, a nutrient, a medicinal substance, etc.) to reach 80% or more (e.g., 80%) (dissolution time) can vary with the type of the active ingredient, but is, for example, at least 2 hours or more, preferably 3 hours or more (e.g., 4 hours or more, 5 hours or more, 6 hours or more, or 7 hours or more).

For the upper limit, the time taken for the dissolution rate of the active ingredient to reach 80% or more (e.g., 80%) is not particularly limited and is, for example, 20 hours or less, 19 hours or less, 18 hours or less, 17 hours or less, 16 hours or less, or 15 hours or less.

For the sustained-release preparation (or granulated material) of the present invention, the time taken for the dissolution rate of the active ingredient to reach 50% can vary with the type of the active ingredient, but is, for example, 20 minutes or more, preferably 30 minutes or more (e.g., 40 minutes or more), more preferably 60 minutes or more (e.g., 80 minutes or more).

For the upper limit, the time taken for the dissolution rate of the active ingredient to reach 50% is not particularly limited and is, for example, 20 hours or less, 19 hours or less, 18 hours or less, 17 hours or less, 16 hours or less, or 15 hours or less.

The method for measuring the dissolution time is not particularly limited and, for example, the Japanese Pharmacopoeia dissolution test method 2 (paddle method) can be used. The Japanese Pharmacopoeia dissolution test method 2 (paddle method) can be performed using 900 mL of distilled water as the test solution at a paddle rotation speed of 50 rpm.

### EXAMPLES

Hereinafter, the present invention will be described in more detail by examples, but the present invention is not limited thereto.

In the following Examples and Comparative Examples, "percentage (%)" and "part" are on a mass basis unless otherwise specified.

The experimental conditions are as follows.

### Average saponification value, average polymerization degree, and viscosity of 4 mass% aqueous solution

These were measured according to JIS K 6726.

### Granulation conditions

Apparatus: Fluid bed granulator MP-01 (manufactured by Powrex)
Powder feed: 500 g
Granulation liquid: aqueous PVA solution 250 g (4 wt%)
Spray speed: 7.5 g/min.
Spray air flow: 35 ml/min.
Inlet air flow: 0.8 m³/min.

### Tableting conditions

Apparatus: Rotary tablet press VIRGO (manufactured by KIKUSUI SEISAKUSHO LTD.)
Tableting pressure: 15 kN
Tablet press rotational speed: 10 rpm
Tablet weight: 200 mg
Tablet diameter: 8 mmϕ

### Evaluation of tablet hardness

The hardness of the obtained tablet was measured using a tablet hardness tester (TBH125 manufactured by ERWEKA). The measurement was performed 6 times, and the average value of the 6 results was used as the measured value.

### Evaluation of sustained-release property

A dissolution test was performed under the test conditions described below. The time taken for the dissolution rate to reach 50% or 80% was measured and calculated. Test method: the Japanese Pharmacopoeia dissolution test method 2 (paddle method) Test fluid: 900 mL of distilled water
Paddle rotation speed: 50 rpm
Samples: tablets shown in Examples and Comparative Examples
Detection wavelengths: UV 444 nm (for riboflavin), UV 325 nm (for nifedipine), UV 270 nm (for theophylline)

The PVA-based polymers (PVA1 to PVA7) used in the following Examples and Reference Examples are listed in Table 1.

The PVAs with the product names listed in Table 1 are PVA products manufactured by JAPAN VAM & POVAL CO., LTD.

**[Table 1]**

| | Product name | Average polymerization degree | Average saponification value (mol%) | Viscosity of 4 mass% aqueous solution (mPa·s) |
|---|---|---|---|---|
| PVA1 | JF-05 | 560 | 98.5 | 5.6 |
| PVA2 | - | 560 | 96.1 | 5.4 |
| PVA3 | JF-10 | 1000 | 98.5 | 10.7 |
| PVA4 | JF-17 | 1700 | 98.5 | 30.1 |
| PVA5 | - | 300 | 99.5 | 3.4 |
| PVA6 | JT-05 | 560 | 94.3 | 5.2 |
| PVA7 | JP-24 | 2400 | 88.0 | 45.2 |

The synthesis methods of PVA2 and PVA5 are shown below.

### Synthesis Example 1

A commercial polyvinyl acetate resin (manufactured by JAPAN VAM & POVAL CO., LTD., JMR-30LL) was dried in vacuo at 100°C for water removal. This was dissolved in methanol to prepare a 50 mass% methanol solution of polyvinyl acetate. For saponification, 500 parts by mass of this solution was heated to 40°C, and 19 parts by mass of a 5.2 mass% methanol solution of sodium hydroxide adjusted to 35°C was added. The mixture was stirred using a propeller-type stirring blade at 300 rpm for 1 minute and then allowed to stand at 40°C for 40 minutes. The obtained gelled product was pulverized and soaked in a mixed solvent composed of 590 parts by mass of methanol, 310 parts by mass of methyl acetate, 14 parts by mass of water, and 46 parts by mass of a 5.2 mass% methanol solution of sodium hydroxide. With gentle stirring, saponification was further performed at 40°C for 1 hour. The reaction mixture was neutralized to a pH of 8 to 9 with a 1 mass% aqueous acetic acid solution. The solid matter was separated from the liquid matter and dried at 60°C for 8 hours to give a PVA-based polymer 2 (PVA2).

The average saponification value of the PVA-based polymer 2 was 96.1 mol% as measured by the method specified in JIS K 6726, and the viscosity of a 4 mass% aqueous solution of the PVA-based polymer 2 was 5.4 mPa·s as measured by the method specified in JIS K 6726.

### Synthesis Example 2

A PVA-based polymer 5 (PVA5) was produced in the same manner as described in Synthesis Example 1, except that saponification was performed under different conditions from those described in Synthesis Example 1 so that the polymerization degree and saponification value described in Table 1 could be achieved.

### Example 1

5 parts by mass of a PVA-based polymer (PVA1) was added to 245 parts by mass of purified water. The mixture was heated to 95°C with stirring for 1 hour to prepare a binder solution (PVA-based polymer concentration: 2 mass%). In a fluid bed granulator, this binder solution was sprayed onto powder particles made by mixing 470 parts by mass of lactose and 25 parts by mass of riboflavin (average particle diameter: 50 µm, total weight of powder particles: 500 g), thus yielding a granular powder [average particle diameter: 155 µm, 1 part by mass of the PVA-based polymer relative to 99 parts by mass of the powder particles (1.0 mass% PVA-based polymer in the granule).

100 parts by mass of the obtained granular powder and 0.5 part by mass of magnesium stearate (St-Mg) were mixed and compressed into tablets. For the obtained tablets, tablet hardness and the time taken for the dissolution rate to reach 50% were measured. The results are shown in Table 2.

### Examples 2 to 6 and 8

In each Example, granulation and tableting were performed in the same manner as described in Example 1, except that the PVA-based polymer and other ingredients listed in Table 2 were used in the amounts described in Table 2. For the obtained tablets, tablet hardness and the time taken for the dissolution rate to reach 50% were measured. The results are shown in Table 2.

### Example 7

Granulation was performed in the same manner as described in Example 1, except that the PVA-based polymer listed in Table 2 and lactose were used in the amounts described in Table 2. The obtained granular powder was mixed with riboflavin and St-Mg in the amounts described in Table 2, and the mixture was compressed into tablets. For the obtained tablets, tablet hardness and the time taken for the dissolution rate to reach 50% were measured. The results are shown in Table 2.

### Example 9

Granulation and tableting were performed in the same manner as described in Example 1, except that the PVA-based polymer and other ingredients listed in Table 3 were used in the amounts described in Table 3. For the obtained tablets, tablet hardness and the time taken for the dissolution rate to reach 80% were measured. The results are shown in Table 3.

### Example 10

Granulation was performed in the same manner as described in Example 1, except that the PVA-based polymer (1) and other ingredients excluding PVA7 and St-Mg listed in Table 3 were used in the amounts described in Table 3. The obtained granular powder was mixed with PVA7 and St-Mg in the amounts described in Table 3, and the mixture was compressed into tablets. For the obtained tablets, tablet hardness and the time taken for the dissolution rate to reach 80% were measured. The results are shown in Table 3.

### Example 11

Granulation was performed in the same manner as described in Example 1, except that the PVA-based polymer (1) and other ingredients excluding PVA7, low-substituted hydroxypropyl cellulose (L-HPC) (NBD-021, manufactured by Shin-Etsu Chemical Co., Ltd.), and St-Mg listed in Table 3 were used in the amounts described in Table 3. The obtained granular powder was mixed with PVA7, L-HPC, and St-Mg in the amounts described in Table 3, and the mixture was compressed into tablets. For the obtained tablets, tablet hardness and the time taken for the dissolution rate to reach 80% were measured. The results are shown in Table 3.

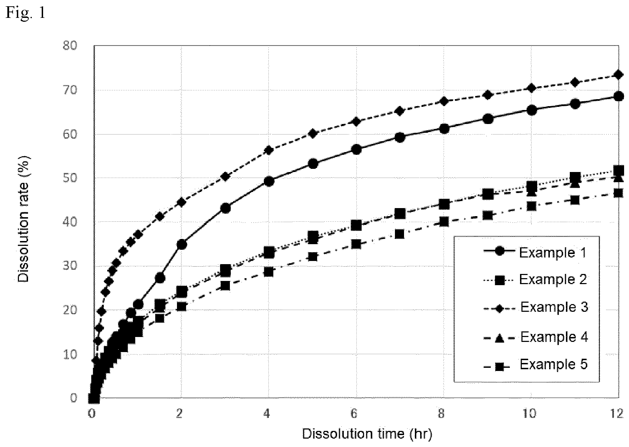

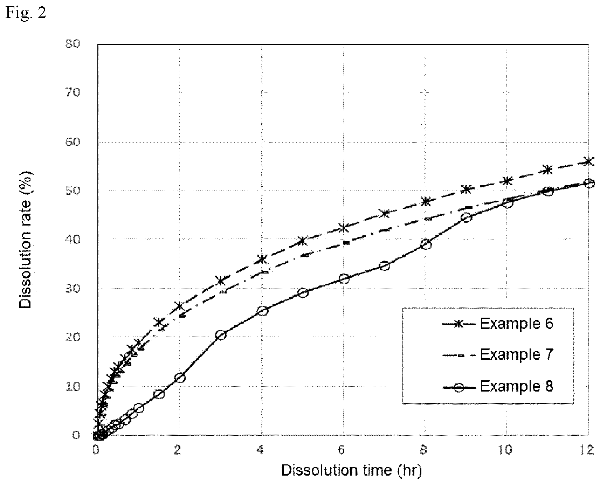

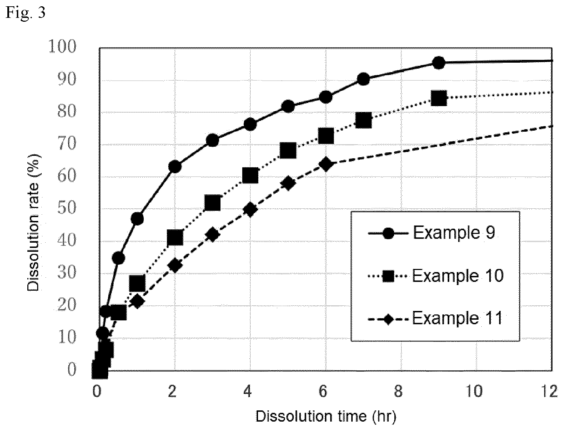

The time course of the dissolution rate in each Example is shown in Fig. 1 (Examples 1 to 5), Fig. 2 (Examples 6 to 8), or Fig. 3 (Examples 9 to 11).

### Reference Example 1

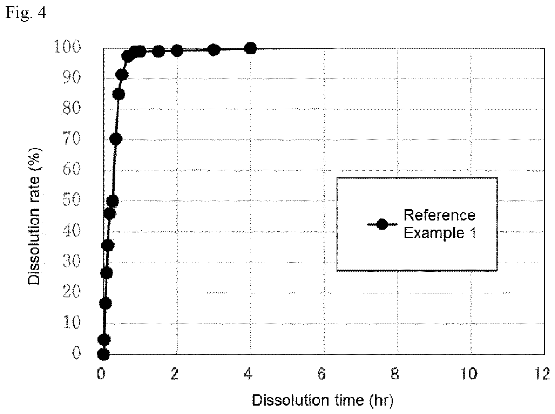

Granulation and tableting were performed in the same manner as described in Example 1, except that the PVA-based polymer and other ingredients listed in Table 4 were used in the amounts described in Table 4. For the obtained tablets, tablet hardness and the time taken for the dissolution rate to reach 50% were measured. The results are shown in Table 4. The time course of the dissolution rate is shown in Fig. 4.

### Reference Example 2

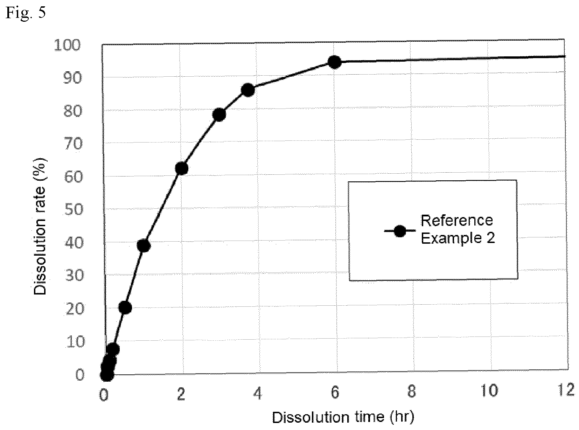

The PVA-based polymer and other ingredients listed in Table 5 were mixed in the amounts described in Table 5, and the mixture was compressed into tablets. For the obtained tablets, tablet hardness and the time taken for the dissolution rate to reach 80% were measured. The results are shown in Table 5. The time course of the dissolution rate is shown in Fig. 5.

**[Table 2]**

| | PVA-based polymer (1) | Amount (parts by mass) | | | | | | | Tablet hardness (N) | Time taken for dissolution rate to reach 50% (hours) |
|---|---|---|---|---|---|---|---|---|---|---|
| | | PVA-based polymer (1) | Lactose | Riboflavin | Nifedipine | Theophylline | St-Mg | Total | | |
| Example 1 | PVA1 | 1 | 94 | 5 | - | - | 0.5 | 100.5 | 115 | 4.0 |
| Example 2 | PVA1 | 3 | 92 | 5 | - | - | 0.5 | 100.5 | 145 | 10.8 |
| Example 3 | PVA2 | 3 | 92 | 5 | - | - | 0.5 | 100.5 | 148 | 3.0 |
| Example 4 | PVA3 | 3 | 92 | 5 | - | - | 0.5 | 100.5 | 152 | 11.8 |
| Example 5 | PVA4 | 2 | 93 | 5 | - | - | 0.5 | 100.5 | 147 | 14.0 |
| Example 6 | PVA5 | 3 | 92 | 5 | - | - | 0.5 | 100.5 | 142 | 8.9 |
| Example 7 | PVA1 | 3 | 92 | 5 | - | - | 0.5 | 100.5 | 142 | 10.9 |
| Example 8 | PVA1 | 1 | 94 | - | 5 | - | 0.5 | 100.5 | 113 | 11.0 |

**[Table 3]**

| | PVA-based polymer (1) | PVA-based polymer (2) | Amount (parts by mass) | | | | | | | Tablet hardness (N) | Time taken for dissolution rate to reach 80% (hours) |
|---|---|---|---|---|---|---|---|---|---|---|---|
| | | | PVA-based polymer (1) | PVA-based polymer (2) | Lactose | Theophylline | L-HPC | St-Mg | Total | | |
| Example 9 | PVA1 | - | 3 | - | 57 | 40 | - | 0.5 | 100.5 | 141 | 4.8 |
| Example 10 | PVA1 | PVA7 | 3 | 37 | 20 | 40 | - | 0.5 | 100.5 | 147 | 7.8 |
| Example 11 | PVA1 | PVA7 | 3 | 37 | - | 40 | 20 | 0.5 | 100.5 | 8.0 | 13.2 |

**[Table 4]**

| | PVA-based polymer (1) | Amount (parts by mass) | | | | | | | | Tablet hardness (N) | Time taken for dissolution rate to reach 50% (hours) |
|---|---|---|---|---|---|---|---|---|---|---|---|
| | | PVA-based polymer (1) | MCC | Lactose | Riboflavin | Nifedipine | Theophylline | St-Mg | Total | | |
| Reference Example 1 | PVA6 | 3 | - | 92 | 5 | - | - | 0.5 | 100.5 | 142 | 0.3 |

**[Table 5]**

| | PVA-based polymer (1) | PVA-based polymer (2) | Amount (parts by mass) | | | | | | Tablet hardness (N) | Time taken for dissolution rate to reach 80% (hours) |
|---|---|---|---|---|---|---|---|---|---|---|
| | | | PVA-based polymer (1) | PVA-based polymer (2) | Lactose | Theophylline | St-Mg | Total | | |
| Reference Example 2 | - | PVA7 | - | 40 | 20 | 40 | 0.5 | 100.5 | 141 | 3.1 |

As shown in the Examples in Tables 2 and 3, granulation using PVA-based polymers having an average saponification value of 96.0 mol% or more was effective in producing tablets having both a high hardness and an excellent sustained-release property.

On the other hand, in the case where a PVA-based polymer having a saponification value of less than 96.0 mol% was used for granulation as in Reference Example 1, the ingredient in the tablets eluted quickly and did not exhibit a sufficient sustained-release property.

In addition, the sustained-release property of the tablets in Examples 9 to 11 was superior to that in Reference Example 2, in which the PVA-based polymer was added without granulation.

### INDUSTRIAL APPLICABILITY

The present invention provides a novel binder. The binder is useful for various applications including the production of granulated materials from powder particles.

## Claims

1. A binder comprising a polyvinyl alcohol-based polymer (1) having an average saponification value of 96.0 mol% or more.

2. A sustained-release base comprising a polyvinyl alcohol-based polymer (1) having an average saponification value of 96.0 mol% or more.

3. A granulated material comprising the binder according to claim 1 or the base according to claim 2.

4. A solid preparation comprising a polyvinyl alcohol-based polymer (1) having an average saponification value of 96.0 mol% or more.

5. The preparation according to claim 4, wherein the preparation is a sustained-release preparation.

6. The preparation according to claim 4 or 5, wherein the preparation is an oral preparation.

7. A solid preparation comprising the granulated material according to claim 3 and a polyvinyl alcohol-based polymer, wherein a 4 mass% aqueous solution of the polyvinyl alcohol-based polymer has a viscosity of 15.0 mPa·s or more as measured according to JIS K 6726, and/or wherein the polyvinyl alcohol-based polymer has an average saponification value of 65.0 to 90.0 mol%.

8. The solid preparation according to claim 7, further comprising a low-substituted hydroxypropyl cellulose.

9. The binder, base, granulated material, or preparation according to any one of claims 1 to 8, wherein the polyvinyl alcohol-based polymer (1) has an average polymerization degree of 200 or more.

10. The binder, base, granulated material, or preparation according to any one of claims 1 to 9, wherein a 4 mass% aqueous solution of the polyvinyl alcohol-based polymer (1) has a viscosity of 3.0 to 30 mPa·s as measured according to JIS K 6726.

11. The granulated material or preparation according to any one of claims 3 to 10, further comprising one or more fillers selected from lactose, mannitol, and crystalline cellulose.

12. A method for producing a granulated material, comprising performing granulation using the binder or base according to any one of claims 1, 2, 9, and 10.

13. A method for granulation using the binder or base according to any one of claims 1, 2, 9, and 10.

14. A method for producing a solid preparation, comprising the step of bringing an aqueous and/or water-based solution containing the binder or base according to any one of claims 1, 2, 9, and 10 into contact with a substance to be mixed with the binder or base.

15. A method for producing a solid preparation, comprising the step of directly compressing a mixture containing the granulated material according to any one of claims 3 and 9 to 11 and a polyvinyl alcohol-based polymer, wherein a 4 mass% aqueous solution of the polyvinyl alcohol-based polymer has a viscosity of 15.0 mPa·s or more as measured according to JIS K 6726, and/or wherein the polyvinyl alcohol-based polymer has an average saponification value of 65.0 to 90.0 mol%.

16. The method according to claim 15, wherein the mixture further contains a low-substituted hydroxypropyl cellulose.
